(19) Europäisches Patentamt
European Patent Office
Office européen des brevets

(11) Veröffentlichungsnummer: **0 065 668**
**B1**

(12) # EUROPÄISCHE PATENTSCHRIFT

(45) Veröffentlichungstag der Patentschrift: **12.12.84**

(21) Anmeldenummer: **82103748.8**

(22) Anmeldetag: **03.05.82**

(51) Int. Cl.³: **C 07 C 127/19,** A 01 N 47/30 // C07C93/14, C07C119/048, C07C79/35

(54) **Harnstoffderivate, ihre Herstellung und Verwendung.**

(30) Priorität: **22.05.81 DE 3120359**

(43) Veröffentlichungstag der Anmeldung: **01.12.82 Patentblatt 82/48**

(45) Bekanntmachung des Hinweises auf die Patenterteilung: **12.12.84 Patentblatt 84/50**

(84) Benannte Vertragsstaaten: **AT BE CH DE FR GB IT LI LU NL SE**

(56) Entgegenhaltungen:
**EP - A - 0 027 965**
**CH - A - 384 282**

(73) Patentinhaber: **CELAMERCK GmbH & Co. KG, Binger Strasse 173, D-6507 Ingelheim am Rhein (DE)**

(72) Erfinder: **Becher, Heinz-Manfred, Dr. Dipl.-Chem., Pfarrer-Heberer-Strasse 5, D-6530 Bingen (DE)**
Erfinder: **Drandarevski, Christo, Dr. Dipl.-Biol., Veit-Stoss-Strasse 17, D-6507 Ingelheim (DE)**
Erfinder: **Lust, Sigmund, Dr. Dipl.-Biol., Klappacher Strasse 2f, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 065 668 B1**

BUNDESDRUCKEREI BERLIN

**Beschreibung**

Die Erfindung betrifft neue Harnstoffderivate mit pestizider Wirkung.

Die erfindungsgemäßen Harnstoffderivate entsprechen der allgemeinen Formel

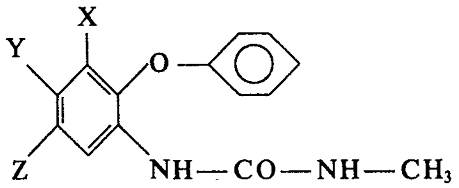

(I)

in der X, Y und Z für Wasserstoff oder Halogenatom stehen, wobei mindestens einer der Substituenten X, Y und Z ein Halogenatom bedeutet. Sind mehrere Halogenatome vorhanden, so können diese gleich oder verschieden sein.

Unter »Halogenatomen« sind hier Fluor, Chlor und Brom zu verstehen. Bevorzugt sind Chlor und Fluor. Hervorzuheben sind vor allem solche Verbindungen der Formel I, die 1 bis 2 Chloratome und ggf. ein Fluoratom im Molekül enthalten.

Die Herstellung der neuen Harnstoffe erfolgt nach an sich bekannten Verfahren

1) durch Umsetzung des Anilins der Formel

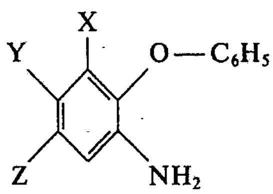

(II)

mit Methylisocyanat oder

2) durch Umsetzung des Isocyanats der Formel

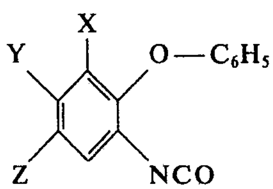

(III)

mit Methylamin.

Die Umsetzungen gemäß 1) und 2) werden vorzugsweise in Gegenwart eines inerten Lösungsmittels bei Temperaturen zwischen der Umgebungstemperatur und der Siedetemperatur des Reaktionsgemischs durchgeführt. Geeignete Lösungsmittel sind z. B. aromatische Kohlenwasserstoffe wie Toluol, Xylol oder Äther wie Dioxan, Tetrahydrofuran.

Die Ausgangsstoffe können nach an sich bekannten Methoden hergestellt werden, z. B. nach dem folgenden Reaktionsschema:

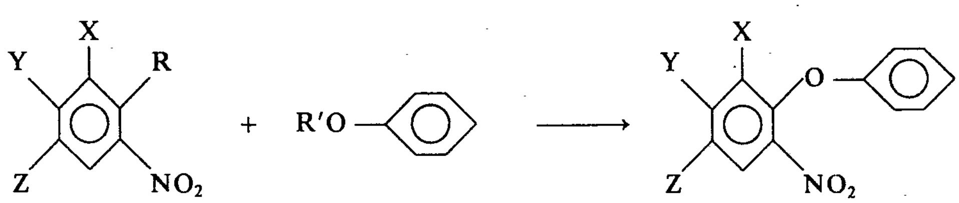

(IV) (V) (VI)

(R = Cl oder F) R' = K oder H)

(VI)

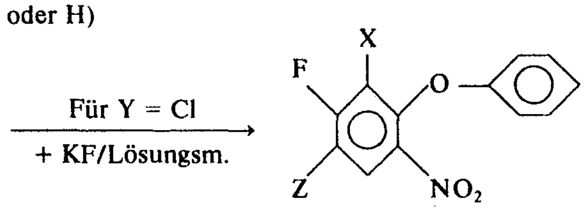


(VIa)

(VI/VIa) ⟶ (II)
kat. Hydrierung oder Metall

(II) ⟶ (III)
+ $COCl_2$

Die Umsetzung von IV und V zu VI wird mit dem Kaliumphenolat in einem Lösungsmittel (Xylol, Xylol plus Dimethylformamid) durchgeführt. Das freie Phenol V eignet sich für die Umsetzung unter den Bedingungen der Phasentransferkatalyse.

Die Verbindungen der Formel I eignen sich als Wirkstoffe für Schädlingsbekämpfungsmittel. Aufgrund ihrer fungiziden Wirkung, insbesondere gegen Pilze der Gattung Tilletia, sind sie besonders gut als Wirkstoffe für Saatgutbeizmittel verwendbar.

Bei dieser Verwendung ist ihr günstiges Umweltverhalten von besonderem Vorteil, denn die erfindungsgemäßen Harnstoffe werden rasch genug zu unbedenklichen Produkten abgebaut, während beispielsweise aus dem für die gleichen Zwecke bekannten Wirkstoff Pentachlornitrobenzol (»Quintozen«) persistente, umweltbelastende Metaboliten entstehen, wie Pentachloranilin, Pentachlorthioanisol. Außerdem besteht bei den erfindungsgemäßen Wirkstoffen nicht die Gefahr der Verunreinigung durch Hexachlorbenzol.

Die gute Wirkung der erfindungsgemäßen Verbindungen geht aus der nachstehenden Tabelle hervor, die Ergebnisse aus dem Tilletia-Sporenkeimtest aufführt (1,6 g Wirkstoff pro 100 kg Weizenkörner):

| Verbindung der Formel I | X | Y | Z | Wirkung in % |
|---|---|---|---|---|
| 1 | Cl | H | H | 99 |
| 2 | H | H | Cl | 99 |
| 3 | Cl | Cl | H | 99 |
| 4 | Cl | F | Cl | 99 |

Die Formulierung der erfindungsgemäßen Verbindungen erfolgt in üblicher Weise mit gebräuchlichen Hilfs- und/oder Trägerstoffen. Der Wirkstoffgehalt kann zwischen etwa 0,1 und 60 Gew.-Prozent betragen. Für Beizmittel wird im allgemeinen ein Wirkstoffgehalt zwischen 0,5 und 20, vorzugsweise zwischen 1 und 10 Gew.-Prozent angewendet. Beispiele für solche Formulierungen sind nachstehend angegeben (in Gewichtsprozent).

A. Beizpuder:

5 % Wirkstoff gemäß der Erfindung,
95 % Talkum oder
5 % Wirkstoff,
5 % roter Pigmentfarbstoff,
90 % Talkum.

Die Beizpuder werden in üblicher Weise als feinvermahlene Mischungen der Bestandteile angewendet. Die Wirkstoffmenge pro 100 kg Saatgut, z. B. Weizen, beträgt dabei etwa 5 bis 50 g.

B. Flüssigkeiten:

Als solche eignen sich Lösungen der erfindungsgemäßen Wirkstoffe in organischen Lösungsmitteln wie Dimethylformamid, Dimethylsulfoxid, Äthylenglykoldimethyläther, Äthylenglykolmonomethyläther, denen gewünschtenfalls ein roter Farbstoff zugesetzt wird. Die Lösungen enthalten im allgemeinen zwischen 1 und 10, vorzugsweise zwischen etwa 2 und etwa 5 Gewichtsprozent Wirkstoff.

Die Herstellung der neuen Verbindungen der Formel I kann entsprechend den nachstehenden Beispielen erfolgen.

## A. Herstellung von Ausgangsstoffen

### 1. 2-Nitro-(mono- bzw. polyhalogen)-diphenyl-äther (VI)

a) 2-Nitro-4,5-dichlor-diphenyl-äther (VI):

Zu der Lösung von 47 g (0,50 Mol) Phenol in 500 ml Xylol werden 32 g 88%iges Ätzkali (≙0,50 Mol KOH) gegeben. Die so erhaltene Mischung wird so lange am Wasserabscheider gekocht, bis sich kein Wasser mehr abscheidet. Man läßt die verbleibende Mischung etwas abkühlen und gibt 113,5 g (0,50 Mol) 2,4,5-Trichlornitro-benzol, 0,5 g Cu-Pulver und 50 ml Dimethylformamid zu. Dieses Gemisch wird 8 h lang am Rückfluß gekocht.

Danach läßt man das Reaktionsgemisch auf Raumtemperatur abkühlen und verrührt es mit 500 ml 5%iger Natronlauge, um nicht umgesetztes Phenol zu entfernen. Dann verrührt man es mit Wasser und engt auf Rückstand ein (zuletzt im Vakuum). Das zurückbleibende Öl wird mit 100 ml Alkohol versetzt und die so erhaltene Mischung im Eisbad gekühlt. Dabei kristallisiert Produkt (VI) aus. Zur Vervollständigung der Fällung werden noch 200 ml Benzin zugegeben. Das Kristallisat wird 2 h später abgesaugt, mit Benzin nachgewaschen und getrocknet.

Ausbeute: 112 g (0,395 Mol; 79 % d. Th.).

Fp.: 74—75°C.

b) Die anderen 2-Nitro-(mono- bzw. dichlor)-diphenyläther (VI) werden nach der gleichen Methode erhalten unter Einsatz der entsprechenden Dichlor- bzw. Trichlornitrobenzole an Stelle des 2,4,5-Trichlornitrobenzols.

2-Nitro-6-chlor-diphenyläther: Öl,

2-Nitro-4-chlor-diphenyläther: Öl,

2-Nitro-3,4-dichlordiphenyläther: Fp. 60—61°C.

c) 2-Nitro-4-chlor-5-fluor-diphenyläther (VI) aus 2-Nitro-4,5-dichlor-diphenyläther (VI):

In die Lösung von 20 g (70,4 mMol) 2-Nitro-4,5-dichlor-diphenyläther (VI) in 70 ml absolutem, frisch im Vakuum destillierten Dimethylsulfoxid werden 11,6 g (200 mMol) wasserfreies Kaliumfluorid eingetragen. Die so erhaltene Mischung wird 8 h lang bei ca. 130°C gerührt. Danach läßt man das Reaktionsgemisch abkühlen und rührt es in 400 ml Wasser ein. Das jetzt vorliegende Gemisch wird 5mal mit je 100 ml Isopropyläther extrahiert. Die vereinigten organischen Phasen werden 2mal mit je 100 ml Wasser ausgeschüttelt, dann mit $MgSO_4$ getrocknet und auf Rückstand eingeengt. Der teilweise kristalline Rückstand wird bei 0—3°C mit ca. 40 ml Isopropyläther verrührt. Das dabei durchkristallisierte Produkt wird abgesaugt.

Ausbeute: 15,4 g (58 mMol; 82 % d. Th.).

Fp.: 64—65°C.

d) Der ölige 2-Nitro-5-fluor-4,6-dichlor-diphenyläther wird nach der unter c) beschriebenen Methode aus 2-Nitro-4,5,6-trichlor-diphenyläther erhalten. Dieser Diphenyläther ist nach der unter a) beschriebenen Methode aus vic. Tetrachlornitrobenzol zugänglich.

### 2. 2-Phenoxy-(mono- bzw. polyhalogen)-aniline (II)

a) 2-Phenoxy-4,5-dichlor-anilin (II):

Die Mischung von 14,2 g (50 mMol) 2-Nitro-4,5-dichlor-diphenyläther (VI), 250 ml Wasser und 5 ml Eisessig wird unter gutem Rühren zum Sieden erhitzt. Nach Erreichen der Siedetemperatur werden 7,5 g Eisenpulver in kleinen Portionen eingetragen. Nach beendeter Eisenzugabe wird noch 6 h lang unter gutem Rühren am Rückfluß gekocht. Anschließend bleibt das Reaktionsgemisch ca. 15 h stehen oder wird in einer Becherzentrifuge geschleudert. Dabei setzen sich die nicht in der wäßrigen Phase gelösten Bestandteile am Gefäßboden ab. Die überstehende wäßrige Phase wird möglichst weitgehend dekantiert. Die zurückbleibende Paste wird mit ca. 150 ml Azeton und ca. 3 g Kieselgur 1 h lang bei Raumtemperatur verrührt. Danach wird das Ungelöste abgesaugt und mit 100 ml Azeton nachgewaschen. Die vereinigten Azetonlösungen werden auf Rückstand eingeengt. Dieser wird mit 100 ml Toluol verrührt. Seine organischen Bestandteile lösen sich im Toluol. Das noch vorhandene Wasser bildet eine eigene Phase und wird abgetrennt. Die organische Phase wird auf Rückstand eingeengt.

Rohausbeute: 12,4 g (48 mMol; 96 % d. Th.).

Das nach Abdestillieren des Lösungsmittels verbliebene Rohprodukt wird im Hochvakuum destilliert.

Reinausbeute: 11,9 g (46,5 mMol; 93 % d. Th.).

Sdp.: ca. 155—160°C/1,07 mbar.

Das destillierte Produkt wird kristallin.

b) Nach der gleichen Methode wurden außerdem folgende Aniline aus den entsprechenden Nitro-Verbindungen dargestellt:

2-Phenoxy-3-chlor-anilin. Öl; Sdp.: ca. 135–139°C/14,4 mbar,
2-Phenoxy-5-chlor-anilin, krist.; Sdp.: ca. 135–138°C/1,07 mbar,
1-Phenoxy-3,4-dichlor-anilin, krist.; Sdp.: ca. 170–175°C/1,07 mbar,
2-Phenoxy-4-fluor-3,5-dichlor-anilin. Öl.

### 3. 2-Phenoxy-(mono- bzw. polyhalogen)-phenyl-isocyanate (III)

a) 2-Phenoxy-4,5-dichlor-phenyl-isocyanat (III):

In die Lösung von 10,16 g (40 mMol) 2-Phenoxy-4,5-dichloranilin (II) in 150 ml Toluol wird bei ca. 5°C HCl-Gas in deutlichem Überschuß unter Rühren eingeleitet. Dabei fällt das entsprechende Hydrochlorid aus. Anschließend werden bei der gleichen Temperatur ca. 10 g Phosgen unter Rühren eingeleitet. Danach wird noch ca. 15 h lang gerührt, wobei man die Temperatur des Reaktionsgemischs langsam auf Raumtemperatur steigen läßt. Anschließend wird unter weiterem Rühren langsam bis auf 95–100°C erhitzt. Dabei entsteht eine klare Lösung. Anschließend wird noch 2 h lang bei 95–100°C gerührt. Danach wird das überschüssige Phosgen durch Durchleiten von Stickstoff bei dieser Temperatur abgetrieben. Die verbleibende Lösung wird dann unter vermindertem Druck eingeengt. Das ölige Produkt verbleibt als Rückstand.
Ausbeute: 11,2 g (40 mMol; 100% d. Th.).

b) Nach der gleichen Methode wurden die unter 2b genannten Aniline (II) ebenfalls zu den entsprechenden Isocyanaten (III) umgesetzt. Die Produkte sind ausnahmslos Öle.

## B. Herstellung der Verbindungen der Formel I

### N-(2-Phenoxy-⟨mono- bzw. polyhalogen⟩-phenyl)-N'-methyl-harnstoffe (I)

### Beispiel 1

#### N-(2-Phenoxy-4,5-dichlor-phenyl)-N'-methyl-harnstoff

In die Lösung von 7,0 g (25 mMol) 2-Phenoxy-4,5-dichlorphenylisocyanat (III) in 70 ml Toluol werden bei Raumtemperatur ca. 3–4 g Methylamin-Gas eingeleitet. Das Reaktionsgemisch bleibt dann ca. 15 h lang bei Raumtemperatur stehen. Dann wird das in dieser Zeit auskristallisierte Produkt (I) abgesaugt, mit etwas Toluol nachgewaschen und getrocknet.
Ausbeute: 6,2 g (20 mMol; 80% d. Th.).
Fp.: 199–201°C.
Nach der gleichen Methode wurden außerdem folgende Harnstoffe (I) aus den entsprechenden Isocyanaten (III) hergestellt:

N-(2-Phenoxy-3-chlor-phenyl)-N'-methyl-harnstoff, Fp.: 168–170°C,
N-(2-Phenoxy-5-chlor-phenyl)-N'-methyl-harnstoff, Fp.: 147–149°C,
N-(2-Phenoxy-3,4-dichlor-phenyl)-N'-methyl-harnstoff, Fp.: 203–205°C,
N-(2-Phenoxy-4-fluor-5-chlor-phenyl)-N'-methyl-harnstoff, Fp.: 123–125°C,
N-(2-Phenoxy-4-fluor-3,5-dichlor-phenyl)-N'-methyl-harnstoff, Fp.: 168–170°C.

### Beispiel 2

#### N-(2-Phenoxy-4,5-dichlorphenyl)-N'-methylharnstoff

Zu der Lösung von 6,35 g (25 mMol) 2-Phenoxy-4,5-dichloranilin in 50 ml wasserfreiem Dioxan werden 2,0 g (35 mMol) Methylisocyanat und 2 Tropfen Triäthylamin gegeben. Die so erhaltene Lösung wird 15 Stunden lang bei 50–60°C gehalten. Danach werden etwa 40 ml Dioxan im Vakuum abdestilliert und der Rückstand mit 50 ml Benzin verrührt. Nach einstündigem Kühlen auf 5–10°C wird das kristalline Produkt abgesaugt, mit 20 ml Benzin gewaschen und getrocknet.
Ausbeute 5,9 g (76% d. Th.).
Fp.: 197–199°C.

**Patentansprüche**

1. Verbindungen der allgemeinen Formel

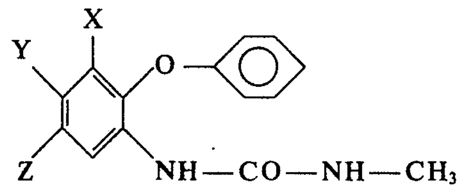

(I)

in der X, Y und Z für Wasserstoff oder Halogenatome stehen, wobei mindestens einer der Substituenten X, Y und Z ein Halogenatom bedeutet.

2. Schädlingsbekämpfungsmittel, gekennzeichnet durch einen Gehalt an mindestens einer Verbindung nach Anspruch 1.

3. Verwendung von Verbindungen nach Anspruch 1 bei der Schädlingsbekämpfung.

4. Verwendung von Verbindungen nach Anspruch 1 als Wirkstoffe in Saatgutbeizmitteln.

5. Verfahren zur Herstellung von Verbindungen nach Anspruch 1, dadurch gekennzeichnet, daß man

a) ein Anilin der Formel

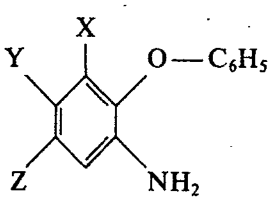

(II)

mit Methylisocyanat oder

b) ein Isocyanat der Formel

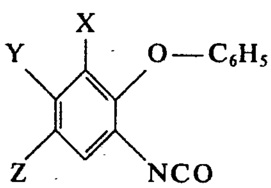

(III)

mit Methylamin umsetzt.

**Claims**

1. Compounds of general formula

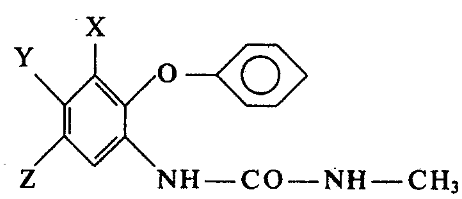

(I)

wherein X, Y and Z represent hydrogen or halogen atoms, with at least one of the substituents X, Y and Z representing a halogen atom.

2. Pesticides, characterised in that they contain at least one compound as claimed in claim 1.

3. Use of compounds as claimed in claim 1 for pest control.

4. Use of compounds as claimed in claim 1 as active ingredients in seed dressings.

5. Process for preparing compounds as claimed in claim 1, characterised in that

a) an aniline of formula

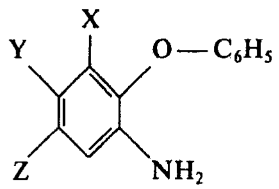

(II)

is reacted with methyl isocyanate or

b) an isocyanate of formula

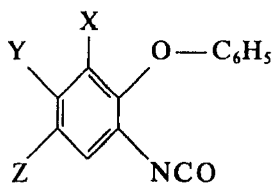

(III)

is reacted with methylamine.

**Revendications**

1. Composés de formule générale

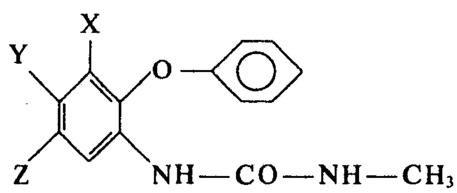

(I)

dans laquelle X, Y et Z représentent l'hydrogène ou des atomes d'halogène, l'un au moins des substituants X, Y et Z signifiant un atome d'halogène.

2. Pesticide caractérisé par une teneur en au moins l'un des composés selon la revendication 1.

3. Application de composés selon la revendication 1 à la lutte contre les parasites.

4. Application de composés selon la revendication 1 comme substance active dans les produits de traitement des semences.

5. Procédé de préparation de composés selon la revendication 1, caractérisé par le fait que l'on fait agir

a) une aniline de formule

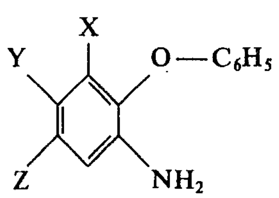

(II)

avec de l'isocyanate de méthyle ou

b) un isocyanate de formule

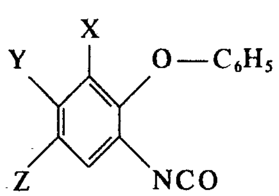

(III)

avec de la méthylamine.